(19) Europäisches Patentamt
European Patent Office
Office européen des brevets

(11) **EP 2 465 412 B1**

(12) **EUROPÄISCHE PATENTSCHRIFT**

(45) Veröffentlichungstag und Bekanntmachung des Hinweises auf die Patenterteilung:
**05.07.2017   Patentblatt 2017/27**

(51) Int Cl.:
*A61B 3/10* *(2006.01)*   *G01B 11/14* *(2006.01)*
*A61B 3/00* *(2006.01)*

(21) Anmeldenummer: **12157249.9**

(22) Anmeldetag: **14.05.2004**

(54) **Verfahren zum Vermessen des vorderen Augenabschnitts**

Method for measuring the front section of the eye

Procédé destinés à mesurer la section avant de l'oeil

(84) Benannte Vertragsstaaten:
**AT BE BG CH CY CZ DE DK EE ES FI FR GB GR HU IE IT LI LU MC NL PL PT RO SE SI SK TR**

(30) Priorität: **22.05.2003   DE 10323920**

(43) Veröffentlichungstag der Anmeldung:
**20.06.2012   Patentblatt 2012/25**

(62) Dokumentnummer(n) der früheren Anmeldung(en) nach Art. 76 EPÜ:
**04739203.0 / 1 624 795**

(73) Patentinhaber: **Carl Zeiss Meditec AG**
**07745 Jena (DE)**

(72) Erfinder:
• **Barth, Roland**
**07743 Jena (DE)**
• **Bergner, Roland**
**07745 Jena (DE)**
• **Voigt, Klaus-Ditmar**
**07745 Jena (DE)**
• **Behrendt, Frank**
**07743 Jena (DE)**

• **Dietzel, Burkhard**
**07616 Bürgel (DE)**
• **Hofmann, Eberhard**
**07646 Bollberg (DE)**
• **Stober, Gert**
**07743 Jena (DE)**
• **Klopfleisch, Peter**
**07751 Jena (DE)**

(74) Vertreter: **Beck, Bernard**
**Carl Zeiss AG**
**Patentabteilung**
**Carl-Zeiss-Promenade 10**
**07745 Jena (DE)**

(56) Entgegenhaltungen:
DE-A1- 4 446 183    DE-A1- 10 108 797
US-A- 5 396 303     US-A- 5 474 548
US-A- 6 082 859     US-A1- 2003 038 921

• DREXLER W ET AL: "Submicrometer precision biometry of the anterior segment of the human eye.", INVESTIGATIVE OPHTHALMOLOGY & VISUAL SCIENCE. UNITED STATES JUN 1997, Bd. 38, Nr. 7, Juni 1997 (1997-06), Seiten 1304-1313, XP009035980, ISSN: 0146-0404

EP 2 465 412 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Anordnung zum Vermessen des vorderen Augenabschnitts. Sie ist insbesondere anwendbar zur Bestimmung der Parameter, die zur Auswahl einer geeigneten Intraokularlinse für eine Kataraktoperation benötigt werden. Außerdem kann sie auch zur Qualitätskontrolle nach erfolgter Implantation einer Intraokularlinse benutzt werden.

Aus DE 198 57 001 ist ein Gerät bekannt, welches zur berührungslosen Messung der Augenlänge, Hornhautkrümmung und Vorderkammertiefe verwendet werden kann. Dabei wird die Achslänge interferometrisch, die Hornhautkrümmung mittels Bildverarbeitung aus Reflexbildern von unter einem bestimmten Winkel auf die Hornhaut projizierten Messmarken und die Vorderkammertiefe aus der Auswertung der Rückstreuung einer spaltförmigen Beleuchtung der Augenlinse bestimmt.

Die beschriebene Messung der Vorderkammertiefe funktioniert bei pseudophaken Augen nicht, da die implantierten Intraokularlinsen (IOL) in der Regel keine streuende Wirkung aufweisen. Die interferometrische Vermessung der Augenachslänge ist bekannt aus "Optical Measurement of the Axial Eye Length by Laser Doppler Interferometry" (Ch. Hitzenberger, Investigative Ophthalmology and Visual Science, Vol. 32, Nr. 3, S. 616, März 1991), auf deren Offenbarung im weiteren Bezug genommen wird.

DE 101 08 797 beschreibt ein Verfahren zur Ermittlung des Durchmessers von Pupille und Iris mit Mitteln der digitalen Bildverarbeitung, wobei unter anderem auch der Winkel zwischen Sehachse und optischer Achse des Auges bestimmt werden kann.

In "Submicrometer Precision Biometry of the Anterior Segment of the Human Eye" (Drexler et all, Investigative Ophthalmology & Visual Science, Vol. 38, Nr. 7, S. 1304, Juni 1997) wird ein Versuchsaufbau beschrieben, mit welchem das vordere Auge interferometrisch vermessen werden kann.

Dabei wird das Auge beim Messvorgang mit einem kollimierten Lichtbündel bestrahlt. Dabei sind die von der Hornhaut und den Linsenflächen reflektierten Lichtanteile, welche auf einen Photodetektor abgebildet werden, relativ schwach. Das Auge muss für die Messung so ausgerichtet werden, dass seine optische Achse mit der Messachse des Gerätes übereinstimmt. Dazu wird dem Patienten entlang einer feststehenden Achse (koaxial) ein kollimiertes Fixierlicht angeboten, welches für das zu vermessende Auge über einen Spiegel eingekoppelt wird. Die Einstellung eines Winkels zwischen Sehachse des Patienten und Messachse des Versuchsaufbaus erfolgt mittels eines Scan-Spiegels.

Bereits bei Abweichung der optischen Achse von der Messachse im Bereich von 1° (z.B. bedingt durch Fixationsprobleme bzw. Nystagmus) können sich die Reflexe von Hornhaut und Linse nicht mehr überlagern, sodass kein Interferenzmesssignal entsteht. Die Messung ist damit sehr empfindlich gegenüber Verkippen des Patientenauges. Weiterhin erscheint das Fixierlicht dem Patienten immer im Unendlichen, was sich als nachteilig erweisen kann.

Die Lage der optischen Achse wird gesucht, indem der Scan-Spiegel in zwei zueinander orthogonalen Richtungen solange verkippt wird, bis alle Messsignale von Hornhaut und Linse zeitgleich nachzuweisen sind. Dieses Verfahren ist extrem zeitaufwendig und führt auch nicht bei allen Patienten zum gewünschten Erfolg. Für den Einsatz in der täglichen klinischen Routine ist dieses Verfahren zu umständlich.

Die Erfindung setzt sich das Ziel die Nachteile des Standes der Technik zu überwinden und eine schnelle und sichere Vermessung des vorderen Augenabschnitts mit interferometrischen Mitteln zu ermöglichen.

Diese Aufgabe wird erfindungsgemäß durch die Merkmale der unabhängigen Ansprüche gelöst, bevorzugte Weiterbildungen sind in den abhängigen Ansprüchen beschrieben.

Die Erfindung wird im folgenden anhand der Figuren beschrieben.

Es zeigen:

Fig. 1            eine schematische Anordung eines bevorzugten Ausführungsbeispiels
Fig. 2            eine schematische Ansicht eines Teils des Interferometerstrahlengangs
Fig. 33 und 4     zwei (unvollständige) Messergebnisse

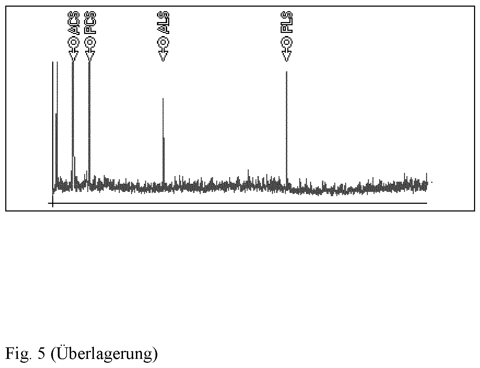

Fig. 5            ein vollständiges Messergebnis

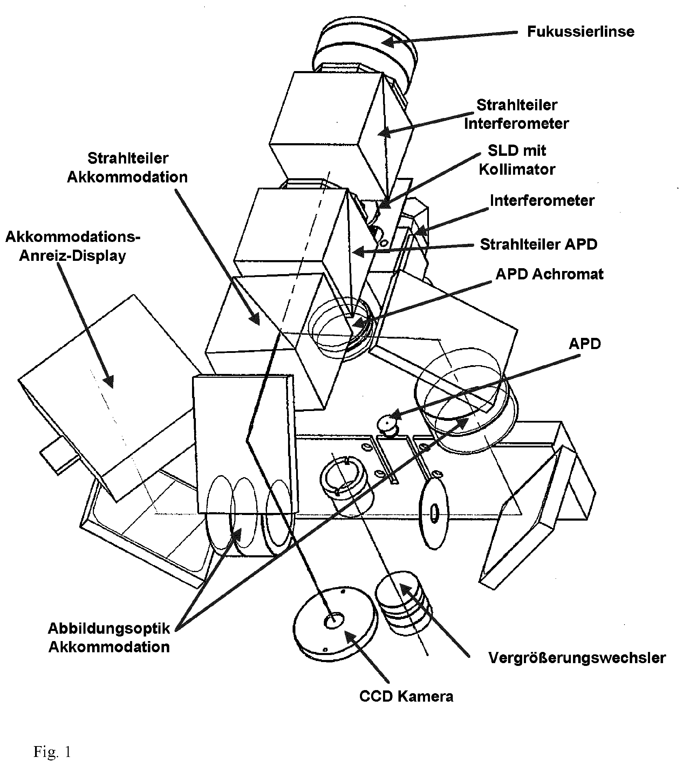

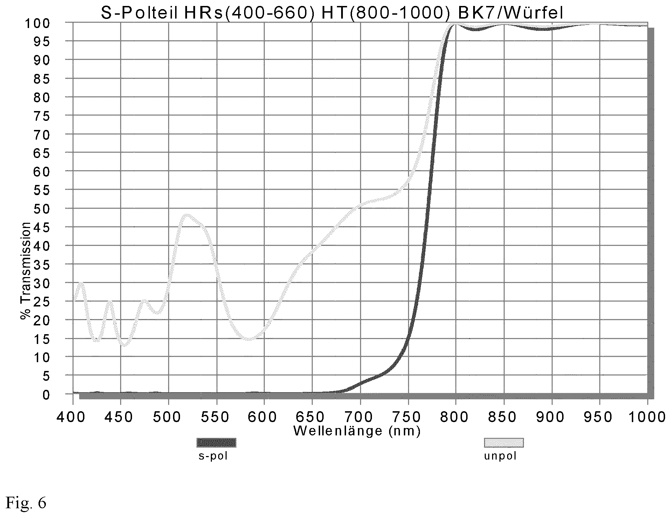

Fig. 6            die Transmissionscharakteristik eines der Strahlteiler aus Fig. 1

In Fig. 1 wird das von einer SLD (Super-Lumineszenz-Diode) emittierte Licht in bekannter Form über ein Interferometer und einen Strahlteiler in Richtung Patientenauge (hier nicht dargestellt) gelenkt. Zwischen Strahlteiler und Auge befindet sich eine Fokussierlinse, welche den Messstrahl in den vorderen Augenabschnitt fokussiert.

Das vom Auge reflektierte Licht wird durch die Fokussierlinse annähernd kollimiert und mittels Strahlteiler APD und APD Achromat auf den hinter einer hier nicht dargestellten Blende liegenden Photodetektor APD (Avalanche Photo Detektor) abgebildet. Ein geringer Lichtanteil (ca. 5%) wird im Strahlteiler transmittiert und mittels geeigneter Optik (Vergrößerungswechsler) auf eine CCD Kamera abgebildet.

Durch gezieltes Defokussieren des Messstrahles mit Hilfe der Fokussierlinse oder Verschieben der Optik relativ zum Patientenauge entlang der optischen Achse der Anordnung werden die Reflexe von Hornhaut und Linse (jeweils Vorder- und Rückseite) unscharf und vergrößert auf den Photodetektor APD abgebildet. Durch die vergrößerte Abbildung der Reflexe wird ein Überlappen der Reflexe in einem größeren Bereich möglich, dadurch verringert sich die Empfindlichkeit gegenüber Verkippung und seitliche Verschiebung des Auges. Unterschiedlich große Lichtintensitäten der Reflexe auf der APD werden vorteilhafterweise durch entsprechende Anpassung der elektronischen Verstärkung ausgeglichen. Die Verstärkung kann dabei automatisch angepasst oder um einen mittleren konstanten Faktor reduziert werden.

Die Empfindlichkeit der Anordnung auf Abweichungen der Augachse von der optischen Achse der Anordnung hängt außerdem vom Verhältnis der Brennweite der Fokussierlinse zu der Brennweite des APD Achromat (APD Linse) ab. Je größer der Quotient beider Brennweiten wird, umso geringer ist diese Empfindlichkeit, so dass auch größere Abweichungen noch ein auswertbares Signal liefern.

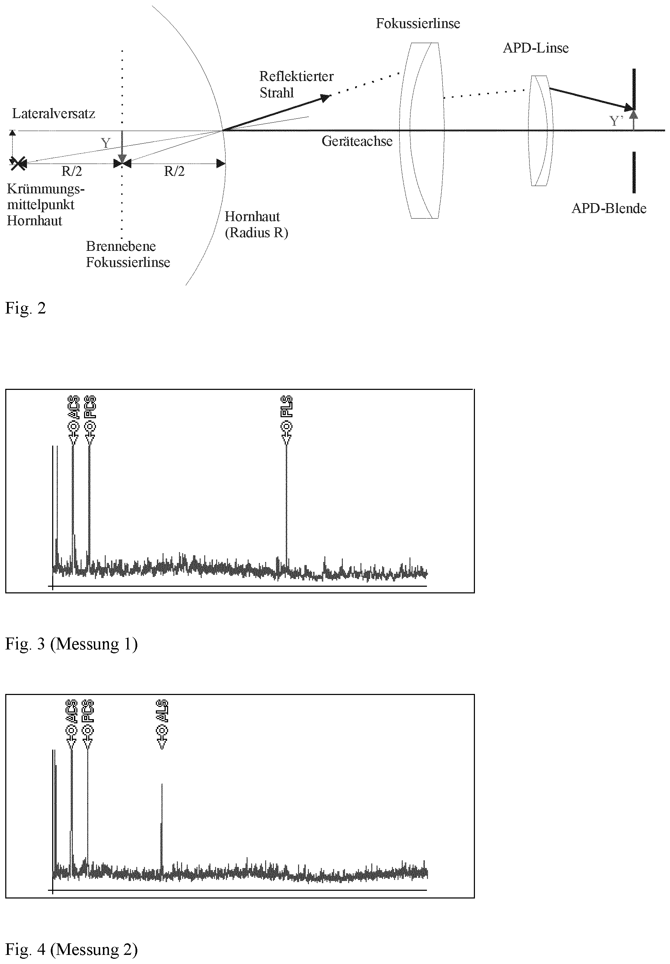

Figur 2 beschreibt diese Abhängigkeit der seitlichen Justiergenauigkeit zum Auge von den Brennweiten der verwendeten Linsen.

Das Auge sei gegenüber der Geräteachse (opt. Achse) lateral versetzt um y.

Der Hauptstrahl muss nach Reflexion an Hornhaut und Durchgang durch Fokussier- und APD-Linse die APD-Blende (Ø=2y') passieren. Die APD-Blende liegt in der Brennebene der APD-Linse.

Zur Ermittlung der Abhängigkeit des zulässigen Lateralversatzes von den Brennweiten der Linsen betrachte man die Abbildung des virtuellen Objektpunktes (Höhe y) in der Brennebene der Fokussierlinse. Für die Abbildung dieses gedachten Objektes auf den Rand der APD-Blende ergibt sich:

$$y' \approx \frac{f_{APD}}{f_{FOK}} y \quad \text{und damit für den Lateralversatz} \quad y \approx \frac{f_{FOK}}{f_{APD}} y'.$$

Wobei:

$f_{FOK}$, $f_{APD}$ : Brennweiten von Fokussier- bzw. APD-Linse
y : zulässiger Lateralversatz
y': halber Blendendurchmesser

Ein optimaler Wert für dieses Verhältnis $f_{FOK}$ : $f_{APD}$ liegt etwa zwischen 1,5 und 4. Für den Fall, dass trotz des vergrößerten Messbereiches nicht alle Signale von Hornhaut und Linse gleichzeitig detektiert werden können, werden mehrere Messungen durchgeführt, deren Ergebnisse geeignet zu einem alle notwendigen Messsignale enthaltenden Gesamtergebnis kombiniert werden.

Eine Möglichkeit für ein solches Verfahren ist in den Fig. 3 bis 5 dargestellt.

Es bedeuten:

ACS - anterior cornea surface (Hornhautvorderfläche)
PCS - posterior cornea surface (Hornhautrückfläche)
ALS - anterior lens surface (Linsenvorderfläche)
PLS - posterior lens surface (Linsenrückfläche)

In Fig. 3 ist kein Reflexsignal der Linsenvorderfläche (ALS) detektiert worden, in Fig. 4 fehlt der Reflex der Linsenrückfläche. In einem ersten Schritt werden die beiden Messreihen auf einen in beiden vorhandenen Reflex, welcher vorzugsweise der Reflex von der Hornhautvorderfläche (ACS) ist, transformiert. Anschließend werden die anderen Werte der Reflexsignale aus beiden Messungen in die Gesamtmessung übernommen. Je nach Qualität der Messungen und Erfordernis können auch mehr als zwei Messungen durchgeführt und in der dargestellten Art und Weise miteinander kombiniert werden. Dabei ist es vorteilhaft wenn aus den verschiedenen Messungen jeweils die zu den Reflexen gehörenden Maximalwerte in die Gesamtmessung übernommen werden.

Es ist für die Durchführung der Messungen günstig, wenn die optische Achse der Messanordnung und die optische Achse des Auges zueinander ausgerichtet sind. Der Durchstoßpunkt der Sehachse durch die Pupille markiert sich durch Reflexion einer koaxialen LED welche in der optischen Achse der Messanordnung angebracht ist (in Fig. 1 aus Gründen der Übersichtlichkeit nicht dargestellt), auf welche der Patient fixiert. Mittels des in DE 101 08 797 beschriebenen Verfahrens kann dann die Abweichung der Sehachse von der optischen Achse des Auges bestimmt werden. Für die Einstellung des zur Messung erforderlichen Winkels wird dann ein in das Gerät integriertes LC-Display (Akkomodations-Anreiz-Display) verwendet, welches über eine Abbildungsoptik Akkomodation sowie einen Strahlteiler Akkomodation in den Strahlengang eingespiegelt wird. Ein Testzeichen (z.B. Kreuz, Punkt o. ä.) wird in Abhängigkeit von dieser bestimmten Abweichung automatisch so verstellt, dass das Patientenauge durch Fixieren auf dieses Zeichen zur Messung ausgerichtet wird. Durch die Fokussierlinse wird die Detektierbarkeit dieser LED entscheidend verbessert, was zur Erhöhung der Messgenauigkeit beiträgt.

Die Einspiegelung der auf dem LC-Display dargestellten Testzeichen im sichtbaren Wellenlängenbereich in den Messstrahlengang (Infrarot) stellt spezifische Anforderungen an den verwendeten Strahlteiler (Strahlteiler Akkommodation).

Hierzu ist eine möglichst hohe Effizienz des einzukoppelnden optischen Signals vom LC-Display in den zum Patientenauge führenden Strahlengang notwendig. Zur Realisierung dieser Forderung wird die Eigenschaft ausgenutzt, dass das vom LC-Display kommende Licht wegen dessen Funktionsprinzips linear polarisiert ist. Der Strahlteiler weist eine nahezu 100%ige Reflexion für s-polarisiertes Licht im VIS-Bereich von 400 bis 650nm auf. Gleichzeitig wird eine möglichst verlustfreie Transmission im nahen Infrarotbereich (830nm...1000nm) realisiert.

Das Schichtdesign erfüllt diese Forderungen für einen Einfallswinkelbereich um 45°. Die eingesetzten Materialien sind bezüglich Brechzahl Substrat, Kittbrechzahl und der Brechzahl der Beschichtungssubstanzen aufeinander abgestimmt. Für diesen speziellen Einsatz wurden folgende Materialien ausgewählt:

Substrat:

BK7 n = 1,64
Kitt n = 1,52
H n = 2,30
L n = 1.48

Das Design besteht aus 23 Wechselschichten H L.

Für vergleichbare Teiler können durch eine geeignete Wahl der Brechzahlen von Substrat und Beschichtungssubstanzen sowie des Einfallswinkels entsprechende Bauelemente gefertigt werden. Durch einen Faktor kann das komplette Schichtsystem bezüglich Kantenlage verschoben werden.

Parameter: hohe Reflexion von 400... 660nm, s-polarisiert

hohe Transmission von 830....1000 nm, unpolarisiert und s-polarisiert

Beispieldaten:

| 1 | TiO2 | 66.28nm |
|---|------|---------|
| 2 | SiO2 | 91.88nm |
| 3 | TiO2 | 102.33nm |
| 4 | SiO2 | 141.87nm |
| 5 | TiO2 | 75.32nm |
| 6 | SiO2 | 156.86nm |
| 7 | TiO2 | 75.32nm |
| 8 | SiO2 | 156.86nm |
| 9 | TiO2 | 75.32nm |
| 10 | SiO2 | 156.86nm |
| 11 | TiO2 | 77.62nm |
| 12 | SiO2 | 130.85nm |
| 13 | TiO2 | 63.37nm |
| 14 | SiO2 | 133.9nm |
| 15 | TiO2 | 49.88nm |
| 16 | SiO2 | 113.15nm |
| 17 | TiO2 | 49.88nm |
| 18 | SiO2 | 113.15nm |
| 19 | TiO2 | 49.88nm |
| 20 | SiO2 | 113.15nm |
| 21 | TiO2 | 64.76nm |

(fortgesetzt)

| 22 | SiO2 | 118.7nm |
| --- | --- | --- |
| 23 | TiO2 | 41.83nm |

Die mit diesem Schichtaufbau erreichten Transmissionswerte für s-polarisiertes und unpolarisiertes Licht sind in Fig. 6 dargestellt.

Vorteilhaft wird das gleiche Display genutzt, um das Auge zum Akkommodieren zu veranlassen. Die Abbildungsoptik Akkommodation bildet in einer definierten Lage des Displays Testzeichen nach unendlich ab. Der Patient kann im akkommodationslosen Zustand die dargebotenen Tests betrachten. Durch (z.B. motorische) Verschiebung des Displays senkrecht zu seiner Ausdehnung erfolgt die Testzeichenabbildung in definierte Abstände vor das Patientenauge (z.B. 40 cm). Der Patient kann die Tests nur scharf sehen, wenn er akkommodiert. Wenn in auf diese Weise erreichbaren verschiedenen Akkomodationszuständen eine Messung im vorderen Augenabschnitt erfolgt, kann der jeweilige Abstand der Linsenvorder- und -rückseiten von der Hornhaut bestimmt werden, womit die für die Akkommodation verantwortlichen Bewegungen bzw. Formveränderungen der Augenlinse nachweisbar sind. Dieses ist insbesondere auch zur Kontrolle der Wirksamkeit akkommodierender Intraokularlinsen verwendbar.

Durch Auswertung der Stellung des Displays, in welcher der Patient gerade noch scharf sehen kann, ist es weiterhin möglich, die Akkommodationsbreite zu bestimmen.

Ein weiterer Vorteil der erfindungsgemäßen Lösung ist die Möglichkeit der Anpassung der Messanordnung an eine eventuell vorhandene Fehlsichtigkeit der Patienten durch entsprechende Verschiebung des LC-Displays bis der Patient die dargestellten Testzeichen scharf sieht.

Für bestimmte Anwendungsfälle (z.B. vor Lasik-OP) ist es wichtig, die Hornhautdicke an mehreren Punkten zu messen. Dazu werden auf dem LC-Display an verschiedenen vorbestimmten Stellen Fixationsreize (z. B. Kreuze, Punkte o. ä.) dargestellt. Dadurch wird der Patient veranlasst, entsprechende Blickwendungen zu vollziehen, so dass die anschließenden Messungen der Hornhautdicke jeweils an dem vorbestimmten Punkt durchgeführt werden. Solche vorbestimmten Punkte können neben der Augenachse auch 1,5mm, 3mm und 4,5 mm von der Achse entfernt sein. Es ist auch möglich, diese Zielpunkte mittels entsprechend angeordneten diskreten LED's zu realisieren.

Die Realisierung der Erfindung ist nicht an das dargestellte Ausführungsbeispiel gebunden, fachmännische Weiterentwicklungen sind möglich ohne den Schutzbereich zu verlassen.

**Patentansprüche**

1.   Verfahren zum Vermessen des vorderen Augenabschnitts eines Patientenauges, wobei mehrere interferometrische Messungen der optischen Grenzflächen Linsenvorderfläche, Linsenrückfläche, Hornhautvorderfläche und Hornhautrückfläche der vorderen Augenabschnittskammer durchgeführt werden, einander entsprechende Messwerte detektiert werden und die Ergebnisse der Messungen durch Anpassung auf jeweils in mehr als einer Messung gemessene sich entsprechende Werte zu einem alle Messwerte von Linse und Hornhaut enthaltenden Gesamtergebnis kombiniert werden.

2.   Verfahren zum Vermessen des vorderen Augenabschnitts nach Anspruch 1,
**gekennzeichnet dadurch,**
**dass** mindestens zwei Messungen durchgeführt werden,
**dass** in einem ersten Schritt zwei Messungen auf einen in beiden Messungen vorhandenen Reflex einer Grenzfläche transformiert werden,
und anschließend die Werte der Reflexsignale der anderen Grenzflächen aus beiden Messungen in das Gesamtergebnis übernommen werden.

3.   Verfahren zum Vermessen des vorderen Augenabschnitts nach Anspruch 2,
**gekennzeichnet dadurch, dass** der in beiden Messungen vorhandene Reflex der Reflex von der Hornhautvorderfläche ist.

4.   Verfahren zum Vermessen des vorderen Augenabschnitts nach Anspruch 1, 2 oder 3,
**gekennzeichnet dadurch, dass** die relative Lage der Augenachse zur optischen Achse der Anordnung bestimmt wird.

5.   Verfahren zum Vermessen des vorderen Augenabschnitts nach Anspruch 4,

**gekennzeichnet dadurch, dass** durch Anbieten eines entsprechend angepassten Fixationsreizes mittels eines Flächenlichtmodulators mit einstellbarer Lichtverteilung eine Ausrichtung der beiden Achsen bewirkt wird.

**Claims**

1. Method for measuring the anterior segment of the eye of a patient, wherein a plurality of interferometric measurements of the optical interfaces of anterior lens surface, posterior lens surface, anterior cornea surface and posterior cornea surface of the chamber of the anterior segment of the eye are carried out, mutually corresponding measurement values are detected and the results of the measurements, by adaptation to mutually corresponding values respectively measured in more than one measurement, are combined to form an overall result containing all the measurement values of lens and cornea.

2. Method for measuring the anterior segment of the eye according to Claim 1,
**characterized**
**in that** at least two measurements are carried out, in that, in a first step, two measurements are transformed to a reflection of an interface that is present in both measurements
and the values of the reflection signals of the other interfaces from both measurements are subsequently accepted into the overall result.

3. Method for measuring the anterior segment of the eye according to Claim 2,
**characterized in that** the reflection present in both measurements is the reflection from the anterior cornea surface.

4. Method for measuring the anterior segment of the eye according to Claim 1, 2 or 3,
**characterized in that** the relative position of the axis of the eye with respect to the optical axis of the arrangement is determined.

5. Method for measuring the anterior segment of the eye according to Claim 4,
**characterized in that** an alignment of the two axes is brought about by offering a correspondingly adapted fixation stimulus by means of a spatial light modulator with adjustable light distribution.

**Revendications**

1. Procédé permettant de mesurer le segment oculaire antérieur de l'oeil d'un patient, dans lequel

   - plusieurs mesures interférométriques des interfaces optiques, à savoir de la surface antérieure du cristallin, de la surface postérieure du cristallin, de la surface antérieure de la cornée et de la surface postérieure de la cornée de la chambre du segment antérieur de l'oeil sont effectuées,
   - des valeurs de mesure correspondant les unes aux autres sont détectées, et
   - les résultats des mesures sont combinés par adaptation respective à des valeurs mesurées qui se correspondent sur plus d'une mesure en un résultat global contenant toutes les valeurs de mesure du cristallin et de la cornée.

2. Procédé permettant de mesurer le segment oculaire antérieur selon la revendication 1,
**caractérisé en ce qu'**au moins deux mesures sont effectuées,
**en ce que**, lors d'une première étape, deux mesures sont transformées en une réflexion d'une interface présente dans les deux mesures,
et enfin, **en ce que** les valeurs des signaux de réflexion des autres interfaces provenant des deux mesures sont reprises dans le résultat global.

3. Procédé permettant de mesurer le segment oculaire antérieur selon la revendication 2,
**caractérisé en ce que** la réflexion présente dans les deux mesures est la réflexion provenant de la surface antérieure de la cornée.

4. Procédé permettant de mesurer le segment oculaire antérieur selon la revendication 1, 2 ou 3,
**caractérisé en ce que** la position relative de l'axe oculaire par rapport à l'axe optique de l'agencement est déterminée.

**5.** Procédé permettant de mesurer le segment oculaire antérieur selon la revendication 4,
**caractérisé en ce qu'**une orientation des deux axes est obtenue par fourniture d'un stimulus de fixation corrélativement adapté au moyen d'un modulateur de lumière en surface ayant une distribution de lumière réglable.

Fig. 1

Fig. 2

Fig. 3 (Messung 1)

Fig. 4 (Messung 2)

Fig. 5 (Überlagerung)

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19857001

- DE 10108797

**In der Beschreibung aufgeführte Nicht-Patentliteratur**

- **CH. HITZENBERGER.** Optical Measurement of the Axial Eye Length by Laser Doppler Interferometry. *Investigative Ophthalmology and Visual Science,* Marz 1991, vol. 32 (3), 616

- **DREXLER.** Submicrometer Precision Biometry of the Anterior Segment of the Human Eye. *Investigative Ophthalmology & Visual Science,* Juni 1997, vol. 38 (7), 1304